(19) Europäisches Patentamt
European Patent Office
Office européen des brevets

(11) Publication number: **0 121 701**
**B1**

(12) **EUROPEAN PATENT SPECIFICATION**

(45) Date of publication of patent specification: **24.08.88**

(51) Int. Cl.$^4$: **C 07 C 131/00**

(21) Application number: **84101743.7**

(22) Date of filing: **20.02.84**

(54) **Process for the production of o-substituted oximes.**

(30) Priority: **04.04.83 US 481687**

(43) Date of publication of application:
**17.10.84 Bulletin 84/42**

(45) Publication of the grant of the patent:
**24.08.88 Bulletin 88/34**

(84) Designated Contracting States:
**DE GB IT NL**

(56) References cited:
**EP-A-0 023 560**

(73) Proprietor: **ALLIED CORPORATION**
**Columbia Road and Park Avenue P.O. Box 2245R**
**(Law Dept.)**
**Morristown New Jersey 07960 (US)**

(72) Inventor: **Mathew, Chempolil Thomas**
**c/o Allied Corporation P.O. Box 2245R**
**Morristown, NJ 07960 (US)**
Inventor: **Ulmer, Harry Edwards**
**c/o Allied Corporation P.O. Box 2245R**
**Morristown, NJ 07960 (US)**

(74) Representative: **Baillie, Iain Cameron et al**
**c/o Langner Parry Isartorplatz 5**
**D-8000 München 2 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 121 701 B1

## Description

This application is a continuation-in-part application of Patent Application Serial Number 481,687, filed April 4, 1983.

Background of the invention

The present invention relates to a novel process for the production of O-substituted oximes. The process is especially useful in the production of O-alkyl oximes.

The classical method of producing O-alkyl oximes involves reacting an oxime with an organohalide (usually bromides or iodides) and an alkoxide, such as sodium methoxide. For example, Dunstan and Goulding have disclosed O-methylation of acetone oxime by reacting acetone oxime with methyl iodide and sodium methoxide. J. Chem. Soc., *91*, 628, 1901. Additionally, it is well known that O-alkyl oximes may be produced by reacting an oxime with sodium metal or a sodium hydride in an inert reaction medium to form an oxime salt which may then be reacted with an alkyl halide to produce the O-alkyl oxime.

EPO Patent Application No. 23,560 (*Linhart et al.*, 1980) discloses two procedures for producing O-alkyl oximes. The first procedure involves a modification of the classical method. The modified procedure is a two step process. Initially, an oxime is converted to salt form by reacting an oxime with an alkoxide. The oxime salt is then purified and reacted with an alkyl bromide or alkyl chloride in an aprotic-dipolar solvent. In practice, *Linhart et al.* employed sodium methoxide for conversion of the oxime to salt form, and then methyl chloride was employed as an alkylating agent.

The second procedure disclosed by *Linhart et al.* accomplished O-alkylation of oximes by reacting oximes with powdered sodium hydroxide and an organohalide in the presence of water in an aprotic-dipolar solvent. It is important to note the definition of aprotic-dipolar solvents. On page 4 of the *Linhart et al.* application, the specification defines such solvents as solvents "whose molecule has a pronounced dipole moment but is devoid of hydrogen atoms capable of hydrogen bonding".

We have unexpectedly discovered a simple one step reaction process for producing O-substituted oximes by reacting an oxime with an organochloride and an alkali-metal hydroxide in a low molecular weight, non-aqueous alcohol reaction medium. The process produces O-substituted oximes, especially O-alkyl oximes, in good yield. The economics of the process are excellent in that expensive alkoxides are avoided, and organochlorides instead of bromides and iodides may be employed.

Brief description of the invention

This invention relates to a process for producing O-substituted oximes. The process comprises reacting oximes of the formula

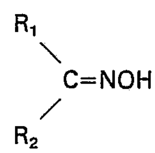

or $R_4{=}NOH$

with an organochloride of the formula $R_3Cl$ and an alkali-metal hydroxide in an alcohol reaction medium wherein $R_1$ and $R_2$ are the same or different and are hydrogen, linear or branched alkyl moieties having 1 to 6 carbon atoms, linear or branched alkenyl moieties having 2 to 6 carbon atoms, alkoxy moieties of 1 to 6 carbon atoms, thioalkyl moieties of 1 to 6 carbon atoms, alkoxy-alkyl moieties of 2 to 8 carbon atoms, substituted or unsubstituted aryl or aralkyl moieties, said aryl or aralkyl moieties being optionally substituted with one or more alkyl moieties having 1 to 8 carbon atoms, one or more alkoxy-alkyl moieties having 2 to 8 carbon atoms, one or more alkoxy moieties of 1 to 6 carbon atoms, one or more thioalkyl moieties of 1 to 6 carbon atoms, and/or one or more cycloalkyl or cycloalkenyl moieties having 3 to 7 carbon atoms; wherein $R_4$ is a substituted or unsubstituted cycloalkyl or cycloalkenyl moiety having 3 to 7 carbon atoms, said cycloalkyl or cycloalkenyl moieties being optionally substituted with one or more alkyl moieties having 1 to 6 carbon atoms, one or more alkoxy-alkyl moieties having 2 to 8 carbon atoms, one or more alkoxy moieties having 1 to 6 carbon atoms and/or one or more thioalkyl moieties having 1 to 6 carbon atoms; wherein $R_3$ is an organic moiety having a primary, secondary or tertiary carbon atom bonded to the chlorine atom of said organochloride; wherein said carbon atom of $R_3$ may optionally be substituted with one or more organic moieties that are inert during said process; wherein said process produces O-substituted oximes of the formula

(I) 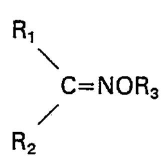 or (II) $R_4{=}NOR_3$

with $R_1$, $R_2$, $R_3$ and $R_4$ being as described above, and with said primary, secondary or tertiary carbon atom of $R_3$ being bonded to the oxygen of said formula I or said formula II.

Detailed description of the invention

We have unexpectedly discovered that reacting oximes having the structure

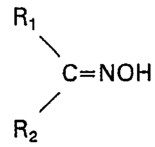 or $R_4{=}NOH$

where $R_1$, $R_2$ and $R_4$ are organic moieties more fully described hereinbelow, with an organochloride, $R_3Cl$, and an alkali-metal hydroxide in an alcohol reaction medium produces O-substituted oximes having the structure

(I) 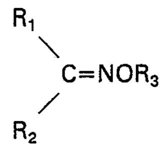 or (II) $R_4{=}NOR_3$

in good yield wherein $R_1$, $R_2$ and $R_4$ are as in the precursor oximes and wherein $R_3$ is as in the organochloride.

In the above organic structures, the organic moieties $R_1$ and $R_2$ are the same or different and said moieties may be hydrogen, linear or branched alkyl of 1 to 6 carbon atoms, linear or branched alkenyl of 2 to 6 carbon atoms, alkoxy of 1 to 6 carbon atoms, thioalkyl moieties of 1 to 6 carbon atoms, alkoxyalkyl moieties of 2 to 8 carbon atoms, substituted or unsubstituted aryl or aralkyl moieties, said substituted aryl or aralkyl moieties being substituted with one or more alkyl moieties of 1 to 8 carbon atoms, substituted or one or more alkoxy-alkyl moieties having 2 to 8 carbon atoms, one or more alkoxy moieties having 1 to 6 carbon atoms, one or more thioalkyl moieties having 1 to 6 carbon atoms and/or one or more cycloalkyl or cycloalkenyl moieties having 3 to 7 carbon atoms.

The organic moiety $R_4$ may be a cycloalkyl or cycloalkenyl moiety having 3 to 7 carbon atoms, either unsubstituted or substituted with one or more groups that are inert during the course of the reaction such as alkyl, aryl, aralkyl, alkoxy, thioalkyl, or alkoxy-alkyl groups.

Illustrative examples of the organic moieties $R_1$ and $R_2$ include, for example, hydrogen, methyl, ethyl, ethoxy, n-propyl, 2-propyl, n-butyl, 2-butyl, t-butyl, benzyl, methylthio, allyl, phenyl, 4-methoxyphenyl, 4-methyl benzyl, 2-methoxy benzyl, 2-phenyl allyl, 3-phenyl allyl, vinyl, 1-methyl vinyl, 1-phenyl allyl, 2-ethoxy ethyl, 4-methylthio phenyl 2-methoxy ethyl, 2-ethoxy methyl or the like.

Illustrative examples of $R_4$ include, for example, cyclohexyl, cyclohex - 2 - enyl, cyclopropyl, cyclopentyl, 4-methyl cyclohexyl, cyclohex - 3 - enyl, 3-methoxy cyclohexyl, 2-methyl cyclopentyl, 2,4 - dimethyl cyclohexyl, cyclopent - 2 - enyl, cycloheptyl, 2-methyl cyclobutyl, 4 - methylthio cyclohexyl, with cyclohexyl and cyclopentyl being the preferred $R_4$ moieties in many embodiments of this invention.

In many preferred embodiments of the invention, the $R_1$ and $R_2$ moieties are different with one of said moieties being hydrogen and one of said moieties being an unsubstituted phenyl moiety. In view of these preferred moieties, it will be appreciated that in many preferred embodiments of this invention, benzaldehyde oxime is a preferred reactant. In other preferred embodiments of the invention, $R_1$ and $R_2$ moieties are the same or different and are alkyl having from about 1 to about 4 carbon atoms, and in the particularly preferred embodiments $R_1$ and $R_2$ are the same or different and are alkyl having from about 1 to about 2 carbon atoms.

Organochlorides, $R_3Cl$, that may be employed in this invention include organochlorides where $R_3$ is an organic moiety having a primary, secondary or tertiary carbon atom bonded to the chlorine atom of said organochloride. The organochlorides may be unsubstituted or substituted with one or more groups that are inert during the course of the reaction such as alkoxy, alkyl, carboxy, alkoxycarbonyl, alkoxy-alkyl, aryl, thioalkyl, aralkyl, cycloalkyl, or the like. In many preferred embodiments of the invention, $R_3$ is unsubstituted and is selected from the group consisting of alkyl moieties having from about 1 to about 4 carbon atoms, alkenyl moieties having from about 2 to about 4 carbon atoms or aralkyl moieties having from about 7 to about 15 carbon atoms. Illustrative examples of these preferred unsubstituted $R_3$ moieties include methyl, ethyl, propyl, vinyl, propenyl and benzyl moieties. Methyl, benzyl and propenyl moieties are especially preferred unsubstituted $R_3$ moieties for this invention, with methyl being the most preferred unsubstituted $R_3$ group. Thus, in many preferred embodiments of this invention, methyl chloride is an organochloride of choice. In other preferred embodiments of this invention, $R_3$ is substituted, and is selected from the group consisting of alkyl having from about 1 to about 4 carbon atoms substituted with a carboxy or alkoxycarbonyl function. Illustrative of these preferred substituted $R_3$ moieties are methyl, ethyl, propyl and butyl substituted with carboxy, methoxycarbonyl, ethoxycarbonyl, propiexy-carbonyl and the like. Methyl substituted with carboxy methoxycarbonyl and ethoxy carbonyl are the most preferred substituted $R_3$ group.

Alkali-metal hydroxides which may be employed in the process include sodium hydroxide, lithium

hydroxide and potassium hydroxide. In view of obvious economic considerations, sodium hydroxide is preferred in many embodiments of this invention. However, potassium and lithium hydroxide work just as well, and they may be employed if other considerations dictate their employment.

The solvent reaction medium for this invention is a low molecular weight alcohol reaction medium. Suitable alcohols include methanol, ethanol, propanol, isopropanol, n-butanol and mixtures thereof. Preferably, however, methanol is the solvent reaction medium of choice for this invention. This preferred embodiment primarily stems from the fact that the alkali-metal hydroxides are more soluble in methanol than in the other solvents.

The process of this invention proceeds by the following general reaction scheme:

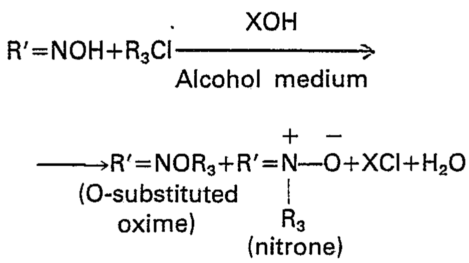


wherein R′ is

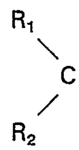 or $R_4$,

with $R_1$, $R_2$ and $R_4$ being as described hereinabove; wherein $R_3$ and said alcohol reaction medium are as described hereinabove, with X being Na, K or Li.

As depicted by the above reaction scheme, the reaction is a simple one-step reaction. The reaction should be conducted under essentially anhydrous conditions. Thus, all reaction ingredients should be dried prior to their employment in this process.

The order of addition of the reactants is not critical and may be varied as desired. However, the preferred order of addition of the reactants is to mix the oxime to be reacted in a solution comprising the alcohol and alkali-metal hydroxide of choice and then to quickly add the organochloride to the solution. The reaction may then be initiated.

Reaction pressures that are suitable for the process of this invention may vary over a wide range. For example, pressures ranging from the autogenous pressure of the reaction mixture up to about 3546 kPa may be employed. More preferably, the reaction pressure should range from ambient to about 1378 kPa. When necessary or desired, the pressure may be boosted *via* the introduction of an inert gas such as nitrogen.

The temperature of the reaction and the reaction time may also be varied over wide ranges. Reaction temperatures may range from about 20°C to about 150°C with about 40°C to about 120°C generally being preferred. Reaction times may vary from about 1 hour to about 48 hours, with 4 hours to about 8 hours generally being preferred when pressure is employed.

It will be appreciated that the reaction pressures, temperatures and times depend upon the reactivity of the oxime to be reacted and upon the reactivity of the organochloride. Thus, higher temperatures, pressures and longer reaction times will be required for organochlorides and oximes that are not very reactive and vice versa. For example, when reacting methyl chloride with benzaldehyde oxime, the following reaction parameters are preferred: 1. temperature: 60°C to 100°C; 2. pressure: 790 kPa to 1134.5 kPa; and 3. time: 4 hours to 8 hours. Those skilled in the art of organic synthesis may easily design reaction conditions that are suitable for specific reactants.

Upon completion of the reaction, the oxime product may be isolated from the reaction mixture by conventional means such as filtration, evaporation, distillation and the like. More specifically, isolation of the O-substituted oxime product may be accomplished by filtering off the chloride salt, evaporating the alcohol reaction medium, dissolving the residue in a hydrocarbon solvent such as toluene, hexane, etc., and then distilling the solution to recover the pure O-substituted oxime.

The O-substituted oximes that may be produced by the process of this invention are valuable as intermediates in the production of O-substituted hydroxylamines, as for example, O-alkyl hydroxylamines such as O-methyl hydroxylamine, O-ethyl hydroxylamine, O-benzyl hydroxylamine, O-allyl hydroxylamine, etc. O-alkyl oximes are valuable as pharmaceutical and agricultural products and intermediates. O-substituted hydroxylamines may be produced by hydrolysis (preferably acid hydrolysis using non-oxidizing acids such as hydrochloric acid, sulfuric acid, phosphoric acid, p-toluene sulfonic acid, etc.) of the O-substituted oximes produced by this invention, wherein the hydrolysis is conducted for a period of

time between about 1 hour and about 25 hours at a temperature between ambient and about 150°C with about 40°C to about 120°C generally being preferred. In some cases, the hydrolysis may be conducted in the oxime product solution without isolation of the O-substituted oxime. For example, O-methyl hydroxylamine, O-allyl hydroxylamine, and O-benzyl hydroxylamine may be produced by hydrolysis of the O-substituted oxime in the product solution. However, best results are generally obtained by isolating the O-substituted oxime and then hydrolyzing said oxime. Hydrolysis of O-methyl benzaldehyde oxime produced in accordance with the process of this invention produces O-methyl hydroxylamine in good yield.

In a preferred embodiment of this invention, O-methyl benzaldehyde oxime is produced. O-methyl benzaldehyde oxime can also be employed as an intermediate in the production of N,O-dimethyl hydroxylamine, a valuable intermediate in the production of pesticides. N,O-dimethyl hydroxylamine may be produced by reacting O-methyl benzaldehyde oxime produced in accordance with this invention with dimethyl sulfate to produce a sulfate salt intermediate; without isolation of the sulfate salt intermediate, the intermediate may then be converted *via* hydrolysis, preferably acid hydrolysis, to produce a product mixture comprising benzaldehyde and N,O-dimethyl hydroxylamine. N,O-dimethyl hydroxylamine may be separated from the product mixture using conventional procedures.

The following Examples more particularly illustrate the process of this invention, but in no way are the Examples to be construed as limitations upon our novel process.

Example 1

Reaction of methyl chloride with benzaldehyde oxime

In a 500 mL autoclave cooled in a dry ice bath was placed a solution of sodium hydroxide pellets (6 g; 0.15 mol) in absolute methanol (150 mL) mixed with benzaldehyde oxime (12.1 g; 0.1 mol). In the meantime, liquid methyl chloride (7.5 g; 0.15 mol) was collected in a dry-ice trap from a cylinder and it was poured quickly into the autoclave and the reactor was closed. After it was allowed to warm up to ambient temperature, the reactor was heated slowly to 100°C with agitation. Thereafter, the reactor was pressurized further to 1135 kPa using dry nitrogen from a cylinder. Heating and stirring continued with occasional re-pressurizing with nitrogen to maintain the pressure at 1135 kPa. At the end of 6 hours, the reactor was allowed to cool and the pressure was released. The reactor-contents which contained a slurry was transferred and the reactor was washed with additional (50 mL) methanol. The slurry (pH 9.9) was neutralized with concentrated HCl (0.6 g) to pH 7. The total sodium chloride was filtered off and the cake washed with more (50 mL) methanol. The total filtrate was analyzed by gas chromatography and then evaporated to remove the methanol completely, and the residue (with small amount of NaCl) was mixed with toluene (200 mL).

Conversion=95.3%; yield: O-methylbenzaldehyde oxime 72.9%, N-methyl nitrone 21.6%, benzaldehyde 0.8%, unreacted benzaldehyde oxime 4.7%.

The solution was washed once with 10% aqueous NaOH solution (50 mL) to remove unreacted benzaldehyde oxime, and the toluene solution carefully distilled under reduced pressure to collect O-methylbenzaldehyde oxime as a colorless liquid (B.P. 52°C; .133 kPa).

The pot residue solidified on cooling to a crystalline solid and this was identified by GC-mass spectrometry to be the dimer of the methyl nitrone. Identification of all the components in the products was established using GC-mass spec (Chemical Ionization) technique.

Example 2

Reaction of ethyl chloride with benzaldehyde oxime

Benzaldehyde oxime (12.1 g; 0.1 mol) and NaOH (6 g; 0.15 mol) in absolute methanol (150 mL) were mixed in a 500 mL autoclave as in Example 1, with liquid ethyl chloride (9.7 g; 0.15 mol) with dry-ice cooling. The reactor was closed and then heated to 80°C. A total pressure of 1066 kPa was provided using nitrogen. Heating and stirring were continued for a total of 6 hours under pressure and the reactor was then cooled to ambient temperature and opened. The slurry was treated with concentrated HCl (1.5 g) to neutralize it from pH 9.7 and worked up as in Example 1.

The product solution was analyzed by gas chromatography which yielded the following product analysis.

| | |
|---|---|
| Benzaldehyde | 1.9% |
| N-ethylbenzaldehyde oxime | 79.5% |
| O-ethyl nitrone | 8.3% |
| Unreacted benzaldehyde oxime | 10.2% |

The identity of all the components was confirmed by GC-mass spectrometry.

On distillation O-ethylbenzaldehyde oxime was collected as a colorless liquid (B.P. 70°C, .266 kPa). The pot residue was identified as the dimer of the ethyl nitrone.

Example 3

Reaction of methyl chloride with benzaldehyde oxime

Benzaldehyde oxime (48.4 g; 0.4 mol) mixed with NaOH (24 g; 0.6 mol) in absolute methanol (200 mL) was reacted with methyl chloride (35 g; 0.69 mol) in the 500 mL pressure reactor as in Example 1.

Temperature was maintained at 180°C and pressure at 1135 kPa during 6 hours of stirring. At the end, concentrated HCl (0.6 g) was added to adjust pH to 7. The slurry was worked up as in Example 1 and the methanolic solution on analysis by gas chromatography showed the following product analysis:

| | |
|---|---|
| Benzaldehyde | 0.6% |
| O-methylbenzaldehyde oxime | 73.0% |
| N-methyl nitrone (as dimer) | 23.8% |
| Unreacted benzaldehyde oxime | 2.6% |

Example 4

Reaction of ethyl chloride with benzaldehyde oxime

In the 500 mL autoclave as in Example 1, benzaldehyde oxime (48.4 g; 0.4 mol) was reacted with methanolic NaOH (24 g; 0.6 mol NaOH in 200 mL solvent) and ethyl chloride (41 g; 0.64 mol). Pressure of 1135 kPa and temperature of 80°C were maintained during 6 hours of stirring. Concentrated HCl (5.9 g) was added to neutralize the slurry to pH 7. After filtration, gas chromatography of the methanolic solution yielded the following product analysis

| | |
|---|---|
| Benzaldehyde | 1.8% |
| O-ethylbenzaldehyde oxime | 83.3% |
| N-ethyl nitrone (as dimer) | 5.6% |
| Benzaldehyde oxime | 5.3% |

Example 5

Reaction of methyl chloride with benzaldehyde oxime

The 500 mL autoclave was charged with benzaldehyde oxime (12.1 g; 0.1 mol) and sodium hydroxide (4.5 g; 0.11 mol) in absolute methanol (150 mL) as in Example 1. Liquid methyl chloride (7.5 g; 0.15 mol) was added and the reactor sealed up and heated with stirring. At 80°C, the autogenous pressure was 411 kPa. Using nitrogen the reactor was pressurized to 1135 kPa and heating and stirring continued for 6 hours. The slurry was worked up and gas chromatography of the methanolic solution yielded the following product analysis:

| | |
|---|---|
| Benzaldehyde | 0.4% |
| O-methylbenzaldehyde oxime | 48.4% |
| N-methyl nitrone (as dimer) | 17.3% |
| Benzaldehyde oxime | 33.9% |

Example 6

Reaction of allyl chloride with benzaldehyde oxime

In a two liter 3-necked flask fitted with a thermometer, reflux condenser with drying tube and dropping funnel was placed benzaldehyde oxime (96.8 g; 0.8 mol) and a solution of sodium hydroxide pellets (48 g; 1.2 mol) in absolute methanol (1200 mL). Allyl chloride (92 g; 1.2 mol) was slowly added (2 minutes) with stirring over a magnetic stirrer and heating mantle. The clear solution was heated at reflux (64°C) and soon turbidity appeared and shortly afterward a slurry resulted. After 7 hours of heating at reflux, it was cooled and neutralized (pH 7) with concentrated HCl (9.6 g) and filtered. The cake was washed with additional methanol (200 mL); gas chromatograph of the total filtrate yielded the following product analysis:

| | |
|---|---|
| Benzaldehyde | 1.0% |
| O-allylbenzaldehyde oxime | 91.6% |
| N-allyl nitrone | 1.3% |
| Benzaldehyde oxime | 6.1% |

The identity of all components was established through GC-mass spec analysis.

The methanolic solution was evaporated (rotovap) under reduced pressure to remove methanol and mixed with toluene (1000 mL). The solution was washed once with 10% NaOH solution (250 mL) to remove unreacted benzaldehyde oxime and then distilled under reduced pressure to collect O-allylbenzaldehyde oxime as a colorless liquid (B.P. 91°C, .133 kPa).

Example 7

Reaction of benzyl chloride with benzaldehyde oxime

A one liter 3-necked flask was fitted with reflux condenser and drying tube and also with a thermometer and a dropping funnel. Benzaldehyde oxime (60.5 g; 0.5 mol) was placed in it with a solution of sodium hydroxide (30 g; 0.75 mol) in methanol (500 mL). With stirring benzyl chloride (95 g; 0.75 mol) was slowly added and a white solid due to NaCl formation was seen in the mixture. It was heated at reflux (67°C) for 2 hours and then cooled and pH adjusted to 7 with concentrated HCl (1.0 g) and filtered. Gas chromatography of the total filtrate yielded the following product analysis:

| | |
|---|---|
| Benzaldehyde | 4.6% |
| O-benzyl benzaldehyde oxime | 94.5% |
| Benzaldehyde oxime | 0.9% |

No measurable amount of N-benzyl nitrone was observed in the product (GC-mass spec analysis).

After removing methanol from the solution under reduced pressure, it was diluted with toluene (400 mL) and washed once with 10% NaOH solution (50 mL). The organic phase was distilled under reduced pressure, first at 1.33 kPa to remove toluene and then at .266 kPa to remove the small amount of benzaldehyde. Subsequently, the pot temperature was raised to 120°C at .133 kPa and nothing distilled over. The pot residue was collected as a colorless liquid, which was O-benzyl benzaldehyde oxime (over 98% pure).

Example 8

Reaction of n-propyl chloride with benzaldehyde oxime

In a 250 mL 3-necked flask fitted with a reflux condenser and drying tube and thermometer were placed benzaldehyde oxime (12.1 g; 0.1 mol) and a solution of sodium hydroxide (6 g; 0.15 mol) in methanol (150 mL). To this was added with stirring 1-chloropropane (n-propyl chloride, 11.7 g; 0.15 mol) and the mixture was heated at reflux (59°C) overnight (20 hours). It was then cooled and pH adjusted to 7 with concentrated HCl (5.75 g). The slurry was filtered and the cake was washed with methanol. The total filtrate yielded the following product analysis:

| | |
|---|---|
| Benzaldehyde | 1.8% |
| O-n-propyl benzaldehyde oxime | 66.4% |
| N-n-propyl nitrone | 7.7% |
| Benzaldehyde oxime | 24.1% |

Example 9

Reaction of iso-propyl chloride with benzaldehyde oxime

The same equipment as in Example 8 was used, and an identical procedure was employed. Benzaldehyde oxime (12.1 g; 0.1 mol), sodium hydroxide (6 g; 0.15 mol) methanol (150 mL) and 2-chloropropane (11.7 g; 0.15 mol) were used. The mixture was heated under reflux (57°C) for 48 hours. On work-up and analysis, gas chromatography of the total filtrate yielded the following product analysis:

| | |
|---|---|
| Benzaldehyde | 2.6% |
| O-isopropyl benzaldehyde oxime | 38.1% |
| N-isopropyl nitrone | 6.5% |
| Benzaldehyde oxime (unreacted) | 52.8% |

Example 10

Reacting of methyl chloride with cyclohexanone oxime

Cyclohexanone oxime (11.3 g; 0.1 mol) was placed in a 500 mL autoclave and a solution of NaOH (6 g; 0.15 mol) in methanol (150 mL) was added to it. Liquid methyl chloride (7.5 g; 0.15 mol) was added to it after cooling the reactor in dry ice. The reactor was then sealed and heated (80°C) with stirring under pressure (1135 kPa). After 6 hours it was cooled and neutralized with concentrated HCl (9.0 g). After filtration and washing with methanol, gas chromatography of the total filtrate yielded the following product analysis:

| | |
|---|---|
| Cyclohexanone | 2.7% |
| O-methyl cyclohexanone oxime | 25.8% |
| N-methyl nitrone | 7.8% |
| Cyclohexanone oxime (unreacted) | 63.7% |

Example 11

Reaction of ethyl chloride with acetone oxime

In a 500 mL autoclave was placed acetoneoxime (7.3 g; 0.1 mol) mixed with a solution of sodium hydroxide (6.0 g; 0.15 mol) in absolute methanol (150 mL). The reactor was cooled in dry ice and liquid ethyl chloride (10 g; 0.15 mol) was added and immediately the reactor was sealed. It was then slowly warmed to 80°C and continued to heat at 80°C with stirring under pressure (1135 kPa) for 6 hours. On cooling the reaction mixture was neutralized with concentrated HCl (5.2 g) and filtered. Gas chromatography of the total filtrate yielded the following product analysis

| | |
|---|---|
| Acetone | 9.2% |
| O-ethyl acetoneoxime | 6.1% |
| Acetoneoxime (unreacted) | 84.5% |

Example 12

Reaction of methyl chloride with methylethylketoxime

In a 500 mL autoclave was placed methylethylketoxime (8.7 g; 0.1 mol) with sodium hydroxide (6.0 g; 0.15 mol) in methanol (150 mL). To the cooled reactor was added liquid methyl chloride (7.5 g; 0.15 mol) and then sealed up and slowly heated. Heating at 80°C under pressure (1135 kPa) with stirring was continued for 6 hours. After cooling, the reaction mixture was neutralized with concentrated HCl (8.2 g) and filtered. The filtrate with cake-washings was analyzed by gas chromatography which yielded the following product analysis:

| | |
|---|---|
| Methylethylketoxime | 4.9% |
| O-methyl methylethylketoxime | 9.9% |
| Methylethylketoxime | 85.2% |

Example 13

Reaction of n-chloropropane with benzaldehyde oxime

In a 500 mL autoclave was placed a mixture of benzaldehyde oxime (12.1 g; 0.1 mol) and a solution of sodium hydroxide (6.0 g; 0.15 mol) in methanol (150 mL). 1-Chloropropane (11.7 g; 0.15 mol) was added and the reactor closed and heated with stirring. Heating (80°C) and stirring continued for 6 hours under pressure (446 kPa). The contents were worked up by neutralizing with concentrated HCl (13 g) and filtered. The filtrate was analyzed by gas chromatography which yielded the following product analysis:

| | |
|---|---|
| Benzaldehyde | 3.8% |
| O-n-propyl benzaldehyde oxime | 68.2% |
| Benzaldehyde oxime | 22.3% |
| N-n-propyl nitrone | 5.7% |

Example 14

Reaction of 2-chloropropane with benzaldehyde oxime

An ethanolic solution of NaOH was prepared by dissolving sodium hydroxide pellets (6.0 g; 0.15 mol) in absolute ethanol (150 mL) and this was placed in a 500 mL autoclave with benzaldehyde oxime (12.1 g; 0.1 mol). 2-chloropropane (11.7 g; 0.15 mol) was weighed into it and the reactor was closed and heated with stirring. At a pressure of 790 kPa and a temperature of 100°C, the reaction was allowed to continue with stirring over 6 hours. After cooling the reactor contents were transferred and neutralized with concentrated HCl (2 g) and filtered and washed with ethanol. The total filtrate was analyzed by gas chromatography which yielded the following product analysis:

| | |
|---|---|
| Benzaldehyde | 3.7% |
| O-isopropyl benzaldehyde oxime | 76.5% |
| N-isopropyl nitrone | 8.0% |
| Benzaldehyde oxime | 11.8% |

Example 15

Reaction of 3-chloropropene with cyclohexanone oxime

A sample of cyclohexanone oxime containing 3.6% water was dried by mixing with toluene and then distilling off the solvent. A portion of this dry cyclohexanone oxime (11.3 g 0.1 mol) was mixed with a solution of sodium hydroxide pellets (6.0 g; 0.15 mol) in methanol (150 mL) in a 250 mL 3-necked flask fitted with drying tube, reflux condensers and a thermometer. Using a dropping funnel 3-chloropropene (11.5 g; 0.15 mol) was added and the mixture heated with stirring at 40—45°C for 16 hours. After the first few minutes turbidity developed and subsequently solid separated.

The slurry was worked up as usual. Gas chromatography of the product solution yielded the following product analysis:

| | |
|---|---|
| Cyclohexanone | 9.1% |
| O-allyl cyclohexanone oxime | 77.0% |
| Cyclohexanone oxime (unreacted) | 13.9% |

Example 16

Reaction of methylchloride with benzaldehyde oxime

Benzaldehyde oxime (12.1 g, 0.1 mol) was placed in a 500 mL autoclave mixed with a solution of potassium hydroxide pellets (8.4 g; 0.15 mol) in absolute methanol (150 mL). The contents were cooled and liquid methyl chloride (7.5 g; 0.15 mol) was quickly added and the reactor sealed. With agitation the reactor was heated (100°C) for 6 hours at a pressure of 928 kPa. The reactor was then cooled and the slurry worked up as usual. Gas chromatography of the product solution yielded the following product analysis:

| | |
|---|---|
| Benzaldehyde | 2.0% |
| O-methylbenzaldehyde oxime | 19.0% |
| N-methyl nitrone | 6.1% |
| Unreacted benzaldehyde oxime | 72.9% |

Example 17

Preparation of O-methyl hydroxylamine (methoxyamine) hydrochloride

A 250 mL 3-neck flask was fitted with reflux condenser and thermometer and a magnetic stirring bar was placed in it. O-methylbenzaldehyde oxime (13.5 g; 0.1 mol) was placed in the flask and then mixed with concentrated (37%) hydrochloric acid (60 g, excess) and n-butanol (70 g). With stirring by a magnetic stirrer, the liquid mixture was heated under reflux for 24 hours. The total reaction mixture was then evaporated to dryness under reduced pressure, with a white solid (8.0 g) being collected.

The identity of the solid as O-methyl hydroxylamine hydrochloride (95.8% yield) was confirmed by treating a solution of a small portion of the solid in water with methyl ethyl ketone and adjusting pH to 6.5 with aqueous NaOH, and then analyzing the mixture for O methylether of methyl ethyl ketoxime, using an authentic sample of the latter.

Example 18

Preparation of O-allylhydroxylamine hydrochloride

The procedure followed was similar to the one in Example 17, except that O-allyl benzaldehyde oxime (16.1 g; 0.1 mol) prepared as in Example 6 was used. It was heated under reflux with 37% concentrated HCl (35 g; excess) and n-butanol (75 g) for 18 hours. The reaction mixture was then evaporated to dryness under reduced pressure (rotovap) and the white solid (9.8 g; yield 90.3%) collected was identified as O-allylhydroxylamine hydrochloride.

The identity of the product was confirmed by treating a portion of the solid with cyclohexanone in aqueous medium at pH 6.5 and characterizing the new product formed as O-allylcyclohexanone oxime which was identical with the product of Example 15.

Example 19

Preparation of N,O-dimethylhydroxylamine

In a 250 mL 3-neck flask fitted with thermometer, reflux condenser and dropping funnel was placed a PTFE-coated magnetic stirring bar. O-methylbenzaldehyde oxime (13.5 g, 0.1 mol) and toluene (100 mL) were added to the reaction flask. Thereafter, with stirring and cooling in a water bath, dimethyl sulfate (12.1 g, 0.1 mol) was slowly added from a dropping funnel to the solution. The solution was heated at reflux (116°C) with stirring for 24 hours and then cooled to ambient temperature and a solution of 10% $H_2SO_4$ (50 mL) was slowly added. The mixture was heated at 50°C for 2 hours, cooled and then the phases separated.

The bottom aqueous phase was extracted once with toluene to remove any of the top phase remaining and then treated with aqueous sodium hydroxide (pH 11). The aqueous solution again extracted with toluene and this solution was analyzed by gas chromatography for N,O-dimethylhydroxylamine using an authentic sample of the product. The analysis confirmed the production of N,O-dimethylhydroxylamine. The yield was 4.6 g (70.5%).

Example 20

Preparation of N,O-dimethylhydroxylamine

The experiment was conducted similarly to the one in Example 19, except that excess of dimethyl sulfate was used in this case. O-methylbenzaldehyde oxime (5 g; 0.037 mol) was mixed with toluene (30 mL) and dimethylsulfate (20 g; 0.16 mol). After heating under reflux (116°C) for 6 hours, the mixture was cooled and then mixed with 10% $H_2SO_4$ solution (50 mL) and heated at 60°C for 4 hours. The top toluene phase was separated and the bottom aqueous phase extracted with more toluene. The aqueous solution was then treated with sodium hydroxide solution (pH 11) with cooling in an ice-water bath, and again extracted thoroughly with toluene. The last toluene extract was analyzed by gas chromatography for N,O-dimethylhydroxylamine. The yield was 2.1 g (93.0%).

The identity of the product was further confirmed by GC-mass spectrometry.

## Claims

1. A process comprising reacting an oxime of the formula

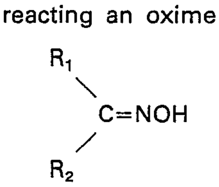 or $R_4$=NOH

with an organochloride of the formula $R_3Cl$ and an alkali-metal hydroxide in a reaction medium; wherein $R_1$ and $R_2$ are the same or different and are hydrogen, linear or branched alkyl moieties having 1 to 6 carbon

atoms, linear or branched alkenyl moieties having 2 to 6 carbon atoms, alkoxy of 1 to 6 carbon atoms, thioalkyl moieties of 1 to 6 carbon atoms, alkoxy-alkyl moieties of 2 to 8 carbon atoms, substituted or unsubstituted aryl or aralkyl moieties, said aryl or aralkyl moieties being optionally substituted with one or more alkyl moieties having 1 to 8 carbon atoms, one or more alkoxy moieties having 1 to 6 carbon atoms, one or more thioalkyl moieties having 1 to 6 carbon atoms, one or more alkoxy-alkyl moieties having 2 to 8 carbon atoms and/or one or more cycloalkyl or cycloalkenyl moieties having 3 to 7 carbon atoms; wherein $R_4$ is a substituted or unsubstituted cycloalkyl or cycloalkenyl moiety having 3 to 7 carbon atoms, said cycloalkyl or cycloalkenyl moieties being optionally substituted with one or more alkyl moieties having 1 to 6 carbon atoms, one or more alkoxy-alkyl moieties having 2 to 8 carbon atoms, one or more alkoxy moieties having 1 to 6 carbon atoms and/or one or more thioalkyl moieties having 1 to 6 carbon atoms; wherein $R_3$ is an organic moiety having a primary, secondary or tertiary carbon atom bonded to the chlorine atom of said organochloride; wherein said carbon atom of $R_3$ may optionally be substituted with one or more organic moieties that are inert during said process; wherein said process produces oximes of the formula

(I) $(R_1)(R_2)C{=}NOR_3$ or (II) $R_4{=}NOR_3$

with $R_1$, $R_2$, $R_3$ and $R_4$ being as described above, and with said primary, secondary or tertiary carbon atom of $R_3$ being bonded to the oxygen of said formula I or said formula II, characterized by carrying out said reaction in a non-aqueous low molecular weight alcohol reaction medium.

2. A process in accordance with claim 1 wherein $R_3$ is an alkyl moiety having 1 to 4 carbon atoms, an alkenyl moiety having 2—4 carbons or an aralkyl moiety, a carboxyalkyl moiety or an alkoxycarbonylalkyl moiety.

3. A process in accordance with claim 1 or 2 wherein said alcohol reaction medium is at least one member selected from the group consisting of methanol, ethanol, isopropanol, propanol and butanol.

4. A process in accordance with any previous claim wherein said process is conducted at a temperature between about 20°C and about 150°C.

5. A process in accordance with any previous claim wherein $R_1$ is hydrogen and $R_2$ is phenyl.

6. A process in accordance with any previous claim wherein said alkali-metal hydroxide is sodium hydroxide.

7. A process in accordance with any previous claim wherein said alcohol reaction medium is methanol.

8. A process in accordance with claim 7 wherein $R_3$ is methyl.

9. A process comprising reacting benzaldehyde oxime with an organochloride of the formula $R_3$—Cl and an alkali-metal hydroxide in an alcohol reaction medium; wherein $R_3$ is an organic moiety having a primary, secondary or tertiary carbon atom bonded to the chlorine atom of said organochloride, said organic moiety being inert during said process; wherein said process produces an O-substituted oxime of the formula

$(H)C{=}NOR_3$ (III)

with $R_3$ as described above and with said primary, secondary or tertiary carbon of $R_3$ being bonded to said oxygen in said formula.

10. A process in accordance with claim 9 wherein $R_3$ is alkyl of 1 to 4 carbons, an alkenyl moiety having 2 to 4 carbon atoms or an aralkyl moiety.

11. The process of any previous claim wherein said oxime of said formula I or II or III is hydrolyzed to produce an O-substituted hydroxylamine of the formula $H_2N$—$OR_3$ wherein the moiety N—$OR_3$ is derived from said oxime of said formulas I or II or III.

**Patentansprüche**

1. Verfahren in dem ein Oxim der Formel

$(R_1)(R_2)C{=}NOH$ or $R_4{=}NOH$

mit einem Organochlorid der Formel $R_3Cl$ und einem Alkalimetallhydroxid in einem Reaktionsmedium umgesetzt wird, wobei $R_1$ und $R_2$ gleich oder verschieden sind und Wasserstoff, lineare oder verzweigte

Alkylanteile mit 1 bis 6 Kohlenstoffatomen, lineare oder verzweigte Aralkylanteile mit 2 bis 6 Kohlenstoffatomen, Alkoxyanteile mit 1 bis 6 Kohlenstoffatomen, Thioalkylanteile mit 1 bis 6 Kohlenstoffatomen, Alkoxy - Alkyl - Anteile mit 2 bis 8 Kohlenstoffatomen, substituierte oder unsubstituierte Aryl- oder Aralkylanteile, die gegebenenfalls mit einem oder mehreren Alkylanteilen mit 1 bis 8 Kohlenstoffatomen, einem oder mehreren Alkoxyanteilen mit 1 bis 6 Kohlenstoffatomen, einem oder mehreren Thioalkylanteilen mit 1 bis 6 Kohlenstoffatomen, einem oder mehreren Alkoxy - Alkyl - Anteilen mit 2 bis 8 Kohlenstoffatomen und/oder einem oder mehreren Cycloalkyl- oder Cycloalkenyl - Anteilen mit 3 bis 7 Kohlenstoffatomen substituiert sind, $R_4$ ein substituierter oder unsubstituierter Cycloalkyl- oder Cycloalkenylanteil mit 3 bis 7 Kohlenstoffatomen ist, der gegebenenfalls mit einem oder mehreren Alkylanteilen mit 1 bis 6 Kohlenstoffatomen, einem oder mehreren Alkoxy - Alkyl - Anteilen mit 2 bis 8 Kohlenstoffatomen, einem oder mehreren Alkoxyanteilen mit 1 bis 6 Kohlenstoffatomen und/oder einem oder mehreren Thioalkylanteilen mit 1 bis 6 Kohlenstoffatomen substituiert ist, $R_3$ ein organischer Anteil ist, der ein primäres, sekundäres oder tertiäres Kohlenstoffatom enthält, das mit dem Chloratom des Organochlorids verbunden ist, das Kohlenstoffatom von $R_3$ gegebenenfalls mit einem oder mehreren organischen Anteilen substituiert ist, die während des Verfahren inert sind, und in dem Verfahren Oxime der Formel

(I) 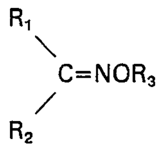 or (II) $R_4{=}NOR_3$

erzeugt werden, in der $R_1$, $R_2$, $R_3$ und $R_4$ die vorstehenden Bedeutungen haben, wobei das primäre, sekundäre oder tertiäre Kohlenstoffatom von $R_3$ mit dem Sauerstoff der Formel I oder der Formel II verbunden ist, dadurch gekennzeichnet, daß die Reaktion in einem nichtwäßrigen Reaktionsmedium auf der Basis eines niedrigmolekularen Alkohols durchgeführt wird.

2. Verfahren nach Anspruch 1, dadurch gekennzeichnet, daß $R_3$ ein Alkylanteil mit 1 bis 4 Kohlenstoffatomen, ein Alkenylanteil mit 2 bis 4 Kohlenstoffatomen oder ein Aralkyl-, Carboxyalkyl- oder Alkoxycarbonylalkylanteil ist.

3. Verfahren nach Anspruch 1 oder 2, dadurch gekennzeichnet, daß als alkoholisches Reaktionsmedium mindestens eines der Glieder der Gruppe ausgewählt wird, die aus Methanol, Ethanol, Isopropanol, Propanol und Butanol besteht.

4. Verfahren nach einem der vorhergehenden Ansprüche, dadurch gekennzeichnet, daß das Verfahren bei einer Temperatur zwischen etwa 20 und etwa 150°C durchgeführt wird.

5. Verfahren nach einem der vorhergehenden Ansprüche, dadurch gekennzeichnet, daß $R_1$ Wasserstoff und $R_2$ Phenyl ist.

6. Verfahren nach einem der vorhergehenden Ansprüche, dadurch gekennzeichnet, daß das Alkalimetallhydroxid Natriumhydroxid ist.

7. Verfahren nach einem der vorhergehenden Ansprüche, dadurch gekennzeichnet, daß das alkoholische Reaktionsmedium Methanol ist.

8. Verfahren nach Anspruch 7, dadurch gekennzeichnet, daß $R_3$ Methyl ist.

9. Verfahren, in dem Benzaldehydoxim mit einem Organochlorid der Formel $R_3$—Cl und einem Alkalimetallhydroxid in einem alkoholischen Reaktionsmedium umgesetzt wird, wobei $R_3$ ein organischer Anteil ist, der ein primäres, sekundäres oder tertiäres Kohlenstoffatom enthält, das mit dem Chloratom des Organochlorids verbunden ist, der organische Anteil während des Verfahrens inert ist und in dem Verfahren ein O-substituiertes Oxim der Formel

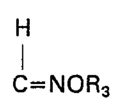 (III)

erzeugt wird, in der $R_3$ die vorstehend angegebene Bedeutung hat und in der das primäre, sekundäre oder tertiäre Kohlenstoffatom von $R_3$ mit dem Sauerstoff der genannten Formel verbunden ist.

10. Verfahren nach Anspruch 9, dadurch gekennzeichnet, daß $R_3$ ein Alkyl mit 1 bis 4 Kohlenstoffatomen, ein Alkenylanteil mit 2 bis 4 Kohlenstoffatomen oder ein Aralkylanteil ist.

11. Verfahren nach einem der vorhergehenden Ansprüche, dadurch gekennzeichnet, daß das Oxim der Formel I oder II oder III zu einem O-substituierten Hydroxylamin der Formel $H_2N$—$OR_3$ substituiert ist, in der der Anteil N—$OR_3$ von dem Oxim der Formel I oder II oder III abgeleitet ist.

## Revendications

1. Procédé consistant à faire réagir une oxime de formule

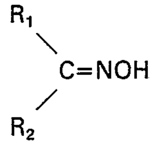 ou $R_4{=}NOH$

avec un organochlorure de formule $R_3$ Cl et un hydroxyde de métal alcalin dans un milieu réactionnel; dans lequel $R_1$ et $R_2$ sont identiques ou différents et sont l'hydrogène, des fractions alkyle linéaires ou ramifiées ayant de 1 à 6 atomes de carbone, des fractions linéaires ou ramifiées d'alcényle ayant de 2 à 6 atomes de carbone, de l'alkoxy à 1 à 6 atomes de carbone, des fractions de thioalkyle à 1 à 6 atomes de carbone, des fractions d'alkoxy-alkyle à 2 à 8 atomes de carbone, des fractions aryle ou aralkyles substituées ou non-substituées, lesdites fractions aryle ou aralkyle étant optionnellement substituées à une ou plusieurs fractions alkyle ayant de 1 à 8 atomes de carbone, une ou plusieurs fractions d'alkoxy ayant de 1 à 6 atomes de carbone, une ou plusieurs fractions d'alkoxy-alkyle ayant de 2 à 8 atomes de carbone et/ou une ou plusieurs fractions de cycloalkyle ou cycloalcényle ayant de 3 à 7 atomes de carbone; dans lequel $R_4$ est un cycloalkyle substitué ou non-substitué ou des fractions de cycloalcényle ayant de 3 à 7 atomes de carbone, lesdites fractions de cycloalkyle ou cycloalcényle étant optionnellement substituées à une ou plusieurs fractions alkyle ayant de 1 à 6 atomes de carbone, une ou plusieurs fractions d'alkoxyalkyle ayant de 2 à 8 atomes de carbone, une ou plusieurs fractions alkoxy ayant de 1 à 6 atomes de carbone et/ou une ou plusieurs fractions de thioalkyle ayant de 1 à 6 atomes de carbone; dans lequel $R_3$ est une fraction organique ayant un atome de carbone primaire, secondaire ou tertiaire lié à l'atome du chlore dudit organochlorure; dans lequel ledit atome de carbone de $R_3$ peut optionnellement être substitué à une ou plusieurs fractions organiques qui sont inertes au cours dudit procédé; dans lequel ledit procédé produit des oximes de formule

(I) 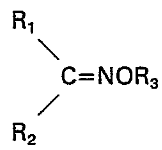 ou (II) $R_4{=}NOR_3$

avec $R_1$, $R_2$, $R_3$ et $R_4$ tels que décrits plus haut, et avec ledit atome de carbone primaire, secondaire ou tertiaire de $R_3$ lié à l'oxygène de ladite formule I ou de ladite formule II, caractérisé en ce que ladite réaction est effectuée dans un milieu réactionnel alcoolique non-aqueux à faible poids moléculaire.

2. Procédé selon la revendication 1 dans lequel $R_3$ est une fraction alkyle ayant de 1 à 4 atomes de carbone, une fraction alcényle ayant 2—4 atomes de carbone, ou une fraction aralkyle, une fraction carboxyalkyle ou une fraction alkoxycarbonylalkyle.

3. Procédé selon l'une des revendications 1 ou 2, dans lequel ledit milieu réactionnel alcoolique est au moins un élément choisi dans le groupe comprenant le méthanol, l'éthanol, l'isopropanol, le propanol et le butanol.

4. Procédé selon l'une quelconque des revendications précédentes dans lequel ledit procédé est conduit à une température comprise entre environ 20°C et environ 150°C.

5. Procédé selon l'une quelconque des revendications précédentes dans lequel $R_1$ est l'hydrogène et $R_2$ est un radical phényle.

6. Procédé selon l'une quelconque des revendications précédentes dans lequel ledit hydroxyde de métal alcalin est de l'hydroxyde de sodium.

7. Procédé selon l'une quelconque des revendications précédentes dans lequel le milieu réactionnel alcoolique est du méthanol.

8. Procédé selon la revendication 7 dans lequel $R_3$ est le radical méthyle.

9. Procédé consistant à faire réagir l'oxime benzaldéhyde avec un organochlorure de formule $R_3$—Cl et un hydroxyde de métal alcalin dans un milieu réactionnel alcoolique; dans lequel $R_3$ est une fraction organique ayant un atome de carbone primaire, secondaire ou tertiaire lié à l'atome de chlore dudit organochlorure, ladite fraction organique étant inerte au cours dudit procédé; dans lequel ledit procédé produit une oxime O-substituée de formule

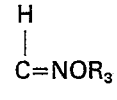 (III)

avec $R_3$ tel que décrit ci-dessus et ledit atome de carbone primaire, secondaire ou tertiaire de $R_3$ lié audit oxygène de ladite formule.

10. Procédé selon la revendication 9 dans lequel $R_3$ est un alkyle de 1 à 4 carbones, une fraction alcényle ayant de 2 à 4 atomes de carbone ou une fraction aralkyle.

11. Procédé selon l'une quelconque des revendications précédentes dans lequel ladite oxime de ladite formule I ou II ou III est hydrolysée pour produire une hydroxylamine O-substituée de formule $H_2N$—$OR_3$ dans laquelle la fraction N—$OR_3$ est dérivée de ladite oxime desdites formules I ou II ou III.